(19) Europäisches Patentamt
European Patent Office
Office européen des brevets

(11) **EP 2 630 974 A1**

(12) **EUROPÄISCHE PATENTANMELDUNG**

(43) Veröffentlichungstag:
28.08.2013 Patentblatt 2013/35

(51) Int Cl.:
*A61L 9/12* *(2006.01)* *A61L 9/01* *(2006.01)*
*G01N 31/22* *(2006.01)*

(21) Anmeldenummer: 12001276.0

(22) Anmeldetag: 27.02.2012

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**
Benannte Erstreckungsstaaten:
**BA ME**

(71) Anmelder: **Symrise AG**
**37603 Holzminden (DE)**

(72) Erfinder: **Rube, Jennifer**
**37671 Höxter (DE)**

(54) **Stabile Raumdüfte mit Nutzungsendpunktanzeige**

(57) Es werden Mischungen offenbart, enthaltend
a. mindestens einen solvatochromen Farbstoff,
b. mindestens ein Lösungsmittel,
c. mindestens einen Duftstoff sowie gegebenenfalls
d. mindestens ein Stabilisierungsmittel.

Die erfindungsgemäßen Mischungen dienen bevorzugt der Beduftung von Räumen und weisen eine Nutzungsendpunktanzeige auf.

EP 2 630 974 A1

**Beschreibung**

Gebiet der Erfindung

Die Erfindung befindet sich auf dem Gebiet der Duftstoffe und betrifft spezielle Mischungen, die Duft- und Farbstoffe enthalten, ein Verfahren zur Nutzungsendanzeige von Beduftungssystemen sowie eine Vorrichtung, die diese Mischungen enthält.

Stand der Technik

Zur Beduftung und Erfrischung von Räumen werden neben Versprühsystemen insbesondere Verdunstungssysteme verwendet. Diese Verdunstungssysteme bestehen in der Regel aus Trägermaterialien, sehr einfachen Behältern sowie sehr speziellen Behältern mit Hilfsmitteln, aus denen die Duftstoffe an die Raumluft abgegeben werden. Seit langer Zeit ist man bestrebt, dem Verbraucher durch ein visuelles Signal anzuzeigen, dass der Vorrat der Duftstoffe verbraucht ist.

In der US 4,128,508 löst man diese Aufgabe durch ein Indikatorsystem enthaltend einen pH-Indikator und eine langsam verdunstende Säure oder Base. Durch die Änderung des pH-Wertes der Duftstoffmischung zeigt die veränderte Farbe des pH-Indikators das Nutzungsende an. WO 03/031966 Al beschreibt ein System enthaltend flüchtige Farben. Durch das Verdunsten der flüchtigen Farbe wird eine Farbänderung erzielt, die den Verbrauch der Duftstoffe signalisiert. Nachteilig an beiden Systemen ist, dass die Farbänderung nicht durch die Duftstoffe selbst hervorgerufen wird, sondern durch Hilfsstoffe. Es ergibt sich zur Erzielung einer verlässlichen Nutzungsendpunktanzeige die Notwendigkeit, die Duftstoffe so auszuwählen, dass diese genau so schnell verdunsten wie die Hilfsstoffe. Die Darstellung eines kreativen und komplexen Duftes wird dadurch unmöglich oder zumindest erheblich erschwert.

GB 2444702 A offenbart nicht wässrige Mischungen enthaltend Halochrome, Duftstoffe, nicht flüchtige Säuren oder Basen sowie hochpolare, schwerflüchtige Lösungsmittel. Nachteilig an einem solchen System sind einerseits die verwendeten Halochrome, welche über sehr reaktive Atome, beispielsweise Halogenatome oder reaktive Gruppen, beispielsweise Aminogruppen, verfügen. Daneben ist die Anwesenheit von Säuren oder Basen erforderlich, welche ein günstiges Milieu für Reaktionen schaffen. Infolge dieser ungünstigen Bedingungen neigen diese Systeme zu vielfältigen Reaktionen, so dass sowohl die Duftstoffe als auch die Halochrome abgebaut werden und bereits nach kurzer Zeit eine Verringerung der Ceruchsintensität bzw. die Bildung von Fehlgerüchen als auch ein Verblassen der Farbe zu beobachten sind. Für eine wirtschaftliche Nutzung ist jedoch eine sehr lange Lager,atabilität der Duftstoffsysteme erforderlich, beispielsweise wegen langer Lagerzeiten beim Vertrieb der Raumdüfte.

Die Aufgabe der vorliegenden Erfindung hat daher in erster Linie darin bestanden, Duftstoffsysteme mit Nutzungsendpunktanzeige zur Verfügung zu stellen, die die eingangs geschilderten Nachteile des Stands der Technik vermeiden. Die Systeme sollten sich dadurch auszeichnen, dass die Endpunkanzeige nicht durch Verringerung der Konzentration eines Hilfsstoffs bewirkt wird. Zudem bestand eine weitere Teilaufgabe darin, besonders lagerstabile Systeme zur Verfügung zu stellen.

**Beschreibung der Erfindung**

Die Aufgabe wird erfindungsgemäß gelöst durch eine Mischung enthaltend

    a. ein, zwei oder mehrere solvatochrome Farbstoffe,
    b. ein, zwei oder mehrere Lösungsmittel
    c. ein, zwei oder mehrere Duftstoffe sowie
    d. optional ein, ein, zwei oder mehrere Stabilisierungsmittel.

Überraschenderweise wurde gefunden, dass Duftsysteme, die neben Lösungsmitteln solvatochrome Farbstoffe enthalten, die oben geschilderte komplexe Aufgabe in vollem Umfang lösen. Insbesondere werden die Nachteile, die mit bekannten indirekten Anzeigemecbanismen verbunden sind, vermieden, denn bei zur Neige gehendem Dunstoffanteil geben die erfindungsgemäßen Systeme ein direktes visuelles Signal ab und zeichnen sich zusätzlich dadurch aus, dass sie über einen sehr langen Zeitraum lagerfähig sind.

**Solvatochrome Farbstoffe**

Solvatochrome Farbstoffe (Komponente a) im Sinne der vorliegenden Erfindung sind solche Farbstoffe, bei denen der Effekt der Solvatochromie auftritt. Solvatochromie ist nach der Definition gemäß Römpp Online (http://www.roempp.com/) "eine Veränderung der Lichtabsorption einer gelösten Substanz (eines Chromophors) in Abhängigkeit vom Lösemittel". Bevorzugt einzusetzende solvatochrome Farbstoffe sind ausgewählt aus Nilrot, Reichardt-Farbstoff, Dimethylaminobenzaldehyd 4-[2-N-substitertes-1,4-hydropyridin-4-ylidine)cthylidcne]cyclohexa-2,5-dien-1-on, 1-(4-bydroxyphcnol)-2,4,6-triphenylpyridinium oder /und der Stoffgruppen der Merocyanine, der roten Pyrazolonfarbstoffe, Azomethinfarbstoffe, Indoanilinfarbstoffe und Pyridinium-N-phenoxidbetaine.

Bevorzugte solvatochorome Farbstoffen sind frei von Halogenatomen und Aminogruppen. Besonders bevorzugte solvatochorome Farbstoffe werden ausgewählt aus Nilrot, Reichardt-Farbstoff oder /und der Stoffgruppe der Merocyanine.

**Lösungsmittel**

Lösungsmittel (Komponente b) sind insbesondere die für Duftstoffe geeignete Lösungsmittel, bevorzugt ausgewählt aus der Gruppe der Alkohole, besonders bevorzugt aus der Gruppe, die gebildet wird von Ethanol, Propylenglycol, Dipropylenglycol, Triethylenglycol, Glycerin und 2-Methyl-1,3 propandiol.

**Duftstoff**

Als Duftstoffe (Komponente c) werden bevorzugt solche Duftstoffe verwendet, die einen angenehmen Geruchseindruck erzeugen. Bevorzugt sind Duftstoffe aus der Gruppe, die gebildet wird von ungesättigten Duftstoffen, der Ketone, der Ester, Ether und Alkohole. Unter ungesättigten Duftstoffe werden im Sinne der Erfindung solche Duftstoffe verstanden, die mindestens eine Doppelbindung im Molekül aufweisen.

In einer speziellen Ausführungsform der Erfindung beträgt das Massenverhältnis der Duftstoffe zu Lösungsmitteln 0,1:1 bis 10:1, bevorzugt 0,2 :1 bis 5:1 und besonders bevorzugt 0,3:1 bis 3:1.

In einer weiteren speziellen Ausführungsform der Erfindung ist der Betrag der Differenz aus dem log pOW der Lösungsmittel und der Duftstoffe mindestens 1. Der log pUW im Sinne der Erfindung wird definiert als der dekadische Logarithmus des n-Oktanol-Wasser-Verteilungskoeffizienten:

$$\log \mathrm{pOW} = \log c_O - \log c_W$$

wobei $c_O$ die Konzentration der Lösungsmittel bzw. der Duftstoffe in der oktanolreichen Phase und $c_W$ die Konzentration der Lösungsmittel bzw. der Duftstoffe in der wasserreichen Phase ist.

Unter dem Betrag der Differenz aus dem log pOW der Lösungsmittel und der Duftstoffe B wird erfindungsgemäß der Wert

$$B = |\log \mathrm{pOW} \, (\text{Lösungsmittel}) - \log \mathrm{pOW} \, (\text{Duftstoffc})|$$

verstanden. Bevorzugt ist B größer oder gleich 1,5, besonders bevorzugt größer oder gleich 2 und insbesondere bevorzugt größer oder gleich 2,5.

In einer weiteren bevorzugten Ausführungsform der Erfindung ist die Verdunstungsrate V der Duftstoffe mindestens doppelt so groß wie die Verdunstungsrate der polaren Lösungsmittel. Unter der Verdunstungsrate V wird erfindungsgemäß der auf die Oberfläche bezogene Massenverlust innerhalb eines definierten Zeitraums unter definierten und identischen Bedingungen verstanden:

$$V = \frac{m\,(t0) - m(t)}{A_P * t}$$

Dabei bedeuten m (t0) die Masse der Probe zu Versuchsbeginn, m(t) die Masse der Probe nach der Verdunstungszeit t, $A_P$ die Oberfläche des Lösungsmittels bzw. der Duftstoffe und t der Zeitraum, in dem die Verdunstung stattgefunden hat. Zur Gewährleistung einer Vergleichbarkeit sind $A_p$, t und m (t0) konstant.

Als Referenz wird die folgende Methode angewendet: 40 mg der des Lösungsmittels oder der Duftstoffe werden auf eine flache Probenpfanne des DVS 1000 der Firma SMS Surface Measurement Systems UK gegeben. Die Massenverluste der Probe werden bei 40°C ermittelt.

Der Membran-Permeationskoeffizient der Duftstoffe ist bevorzugt mindestens doppelt so groß wie der Membran-Permeationskoeffizient der polaren Lösungsmittel. Unter dem Membran-Permeatiooskoeffizienten M wird erfindungsgemäß der flächenbezogene Massenstrom der Lösungsmittel bzw. der Duftstoffe durch die semipermeable Membran verstanden.

$$M = \frac{m\,(t0) - m(t)}{A_M * t}$$

Darin bedeuten m (t0) die Masse der Probe zu Versuchsbeginn, m(t) die Masse der Probe nach der Verdunstungszeit L $A_M$ die Oberfläche der Membran und t der Zeitraum, in dem die Verdunstung stattgefunden hat. Zur Gewährleistung einer Vergleichbarkeit sind $A_M$, t und m (t0) konstant.

Als Referenz zur Bestimmung des Membran-Permeationskoeffizienten M wird die folgende Methode angewendet: 20g Lösungsmittel bzw. Duftstoffe werden in eine Petrischale aus Glas gegeben. Die semipermeable Membran wird so auf den Rand der Petrischale aufgeklebt, dass die Öffnung der Petrischale vollständig mit der Membran bedeckt ist und der Klebstoff die Anordnung so abdichtet, dass das Lösungsmittel bzw. die Duftstoffe nicht seitlich entweichen können. Nach 2 Wochen Lagerung bei 25 °C wird der Massenverlust und M gemäß der Gleichung bestimmt

Der pH-Wert der erfindungsgemäßen Mischung ist vorzugsweise im neutralen Bereich einzustellen. Bevorzugt beträgt der pH-Wert der erfindungsgemäßen Mischung etwa 5 bis etwa 9 und besonders bevorzugt 6 bis 8.

Die Mischung kann optional Stabilisierungsmittel enthalten, Unter Stabilisierungsmitteln werden erfindungsgemäß bevorzugt Lichtschutzmittel, Antioxidantien und Emulgatoren verstanden.

In einer weiteren Ausführungsform der Erfindung liegt die erfindungsgemäße Mischung in einem Behälter vor, der eine semipermeable Membran aufweist oder aus einer semipermeablen Membran besteht.

Die Erfindung betrifft ferner ein Verfahren zur Nutzungsendpunktsanzcige von Duftstoffsystemen, bei dem man Duftstoffe mit Lösungsmitteln und solvatochromen Farbstoffen und gegebenenfalls Stabilisatoren vermischt und der Luft aussetzt, wobei ein Farbumschlag im Bereich des sichtbaren Lichtes stattfindet, sobald eine hinreichende Menge an Lösungsmittel oder Duftstoff an die Luft abgegeben worden ist. Bevorzugt lassen sich die Duftstoffsysteme, bei dem man Duftstoffe mit Lösungsmitteln und solvatochromen Farbstoffen und gegebenenfalls Stabilisatoren vermischt über einen längeren Zeitraum lagern, bevor Lösungsmittel oder Duftstoff an die Luft abgegeben werden. Der Lagerzeitraum kann einen mindestens einen Tag, bevorzugt mindestens eine Woche, weiter bevorzugt mindestens einen Monat und insbesondere bevorzugt mindestens ein Jahr betragen. Die Temperatur während der Lagerung kann zwischen 0 und 50°C erfolgen, bevorzugt zwischen 15 und 30°C. Es ist dabei nicht notwendig eine konstante Temperatur einzuhalten. In der bevorzugten Ausführung der Erfindung wird der Geruch der Mischung oder/und die Farbe während der Lagerung nicht verändert. Zur Beurteilung einer Farb- oder/und einer Geruchsveränderung kann insbesondere der Duo-Trio Test gemäß DIN EN ISO 10399:2010-06 herangezogen werden.

**Patentansprüche**

1. Mischungen enthaltend

   a. mindestens einen solvatochromen Farbstoff
   b. mindestens ein Lösungsmittel,
   c. mindestens einen Duftstoff sowie gegebenenfalls
   d. mindestens ein Stabilisierungsmittel.

2. Mischungen nach Anspruch 1, **dadurch gekennzeichnet, dass** sie solvatochrome Farbstoffe (Komponente a) enthalten, die frei von Halogen- und frei von Aminogruppen sind.

**3.** Mischungen nach mindestens einem der Ansprüche 1 und/oder 2, **dadurch gekennzeichnet, dass** sie solvatochrome Farbstoffe (Komponente a) enthalten, die ausgewählt sind aus der Gruppe, die gebildet wird von Nilrot, Rcichardt-Farbstoff, Dimethylaminobenzatdehyd 4-[2-N-substitertes-1,4-hydropyridin-4-ylidine)ethylidene]cyclohaxa-2,5-dien-1-on, 1-(4-hydroxyphenol)-2,4,6-triphenylpyridinium und deren Gemischen.

**4.** Mischungen nach mindestens einem der Ansprüche 1 und/oder 2, **dadurch gekennzeichnet, dass** sie solvatochrome Farbstoffe (Komponente a) enthalten, die ausgewählt sind aus der Gruppe die gebildet wird von Merocyaninen, roten Pyrazolonfarbstoffen, Azomethinfarbstoffen, Indoanilinfarbstoffen, Pyridinium-N-phenoxidbetainen und deren Gemischen.

**5.** Mischungen nach mindestens einem der Ansprüche 1 bis 4, **dadurch gekennzeichnet, dass** sie Lösungsmittel (Komponente b) enthalten, die ausgewählt sind aus der Gruppe, die gebildet wird von Ethanol, Propylenglycol, Dipropylenglycol, Triethylenglycol, Glycerin, 2-Methyl-1,3-propandiol und deren Gemischen.

**6.** Mischungen nach mindestens einem der vorhergehenden Ansprüche 1 bis 5, **dadurch gekennzeichnet, dass** sie Duftstoffe (Komponente c) enthalten, die ausgewählt sind aus der Gruppe, die gebildet wird von ungesättigen Duftstoffen, Ketonen, Estern, Ethern und Alkoholen.

**7.** Mischungen nach mindestens einem der vorhergehenden Ansprüche 1 bis 6, **dadarch gekennzeichnet, dass** das Massenverhältnis der Duftstoffe (Komponente c) zu Lösungsmitteln (Komponente b) 0,1:1. bis 10:1 beträgt.

**8.** Mischungen nach mindestens einem der vorhergehenden Ansprüche 1 bis 7, **dadurch gekennzeichnet, dass** der Betrag der Differenz aus dem log pOW der Lösungsmittel (Komponente b) und der Duftstoffe (Komponente c) mindestens 1 beträgt.

**9.** Mischungen nach mindestens einem der vorhergehenden Ansprüche 1 bis 8, **dadurch gekennzeichnet, dass** sie Stabilisierungsmittel (optionale Komponente d) enthalten, die ausgewählt sind aus der Gruppe, die gebildet wird von Lichtschutzmitteln, Antioxidationsmitteln, Emulgatoren und deren Gemischen.

**10.** Mischungen nach mindestens einem der vorhergehenden Ansprüche 1 bis 9, **dadurch gekennzeichnet, dass** sie polare Lösungsmittel und Duftstoffe enthalten und die Verdunstungsrate der Duftstoffe mindestens doppelt so groß wie die Verdunstungsratc der polaren Lösungsmittel ist.

**11.** Mischungen nach Anspruch 10, **dadurch gekennzeichnet, dass** der Membran-Permeationskoeffizient der Duftstoffe mindestens doppelt so groß wie der Membran-Permeationskoeffizient der polaren Lösungsmittel ist.

**12.** Mischungen nach einem der vorhergehenden Ansprüche 1 bis 11, **dadurch gekennzeichnet, dass** sie in einem Behälter vorliegen, der eine semipermeable Membran aufweist oder aus einer semipermeablen Membran besteht.

**13.** Mischungen nach Anspruch 12 **dadurch gekennzeichnet, dass** der Behälter mit Ausnahme der semipermeablen Membran aus einem synthetischen Kunststoff besteht.

**14.** Verfahren zur Nutzungsendpunktsanzeige von Duftstoffsystemen, bei dem man Duftstoff mit Lösungsmitteln und solvatochromen Farbstoffen und gegebenenfalls Stabilisatoren vermischt und der Luft aussetzt, wobei ein Farbumschlag im Bereich des sichtbaren Lichtes stattfindet, sobald eine hinreichende Menge an Lösungsmittel oder Duftstoff an die Luft abgegeben worden ist.

**15.** Behälter mit einem Verschluss aus einer semipermeablen Membran, der eine Mischung nach Anspruch 1. enthält.

Europäisches Patentamt

European Patent Office

Office européen des brevets

## EUROPÄISCHER RECHERCHENBERICHT

**Nummer der Anmeldung**

EP 12 00 1276

### EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X | DE 10 2009 058078 A1 (ARIS HANDELS GMBH [DE]) 8. Juli 2010 (2010-07-08) * Zusammenfassung * * Absätze [0007] - [0013], [0024] - [0026] * ----- | 1-15 | INV. A61L9/12 A61L9/01 G01N31/22 |
| X | EP 0 309 173 A2 (JOHNSON & SON INC S C [US]) 29. März 1989 (1989-03-29) * Seite 3, Zeile 56 - Seite 5, Zeile 11 * ----- | 1,5,6,9, 12,14,15 | |
| X | WO 2009/044363 A2 (OREAL [FR]; CASSIN GUILLAUME [FR]; NGUYEN QUANG LAN [FR]; SIMONNET JEA) 9. April 2009 (2009-04-09) * Seiten 13-14,23 * ----- | 1,9 | |
| X | WO 2011/042222 A1 (HENKEL AG & CO KGAA [DE]; SUNDER MATTHIAS [DE]; MEIER FRANK [DE]; FREE) 14. April 2011 (2011-04-14) * Seite 4; Anspruch 1 * ----- | 1 | |
| | | | RECHERCHIERTE SACHGEBIETE (IPC) A61L G01N |

Der vorliegende Recherchenbericht wurde für alle Patentansprüche erstellt

| Recherchenort | Abschlußdatum der Recherche | Prüfer |
|---|---|---|
| München | 27. August 2012 | Varga, Viktoria |

EPO FORM 1503 03.82 (P04C03)

# EP 2 630 974 A1

**ANHANG ZUM EUROPÄISCHEN RECHERCHENBERICHT
ÜBER DIE EUROPÄISCHE PATENTANMELDUNG NR.**

EP 12 00 1276

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

27-08-2012

| Im Recherchenbericht angeführtes Patentdokument | Datum der Veröffentlichung | Mitglied(er) der Patentfamilie | Datum der Veröffentlichung |
|---|---|---|---|
| DE 102009058078 A1 | 08-07-2010 | KEINE | |
| EP 0309173 A2 | 29-03-1989 | EP 0309173 A2<br>GB 2209942 A<br>US 4824827 A | 29-03-1989<br>01-06-1989<br>25-04-1989 |
| WO 2009044363 A2 | 09-04-2009 | FR 2921559 A1<br>WO 2009044363 A2 | 03-04-2009<br>09-04-2009 |
| WO 2011042222 A1 | 14-04-2011 | DE 102009045482 A1<br>EP 2453929 A1<br>US 2012193443 A1<br>WO 2011042222 A1 | 14-04-2011<br>23-05-2012<br>02-08-2012<br>14-04-2011 |

EPO FORM P0461

Für nähere Einzelheiten zu diesem Anhang : siehe Amtsblatt des Europäischen Patentamts, Nr.12/82

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 4128508 A
- WO 03031966 A1

- GB 2444702 A